# EUROPEAN PATENT SPECIFICATION

(11) **EP 3 375 861 B1**
(45) Date of publication and mention of the grant of the patent: **29.07.2020**
(21) Application number: 15908391.4
(22) Date of filing: 25.12.2015
(51) Int. Cl.: A01P 3/00, A01N 63/10, C12R 1/01, C12N 1/20

(54) **STRAIN OF PAENIBACILLUS MUCILAGINOSUS FOR USE AS A FERTILISER, FOR GROWTH STIMULATION, AND FOR THE PROTECTION OF PLANTS FROM FUNGAL DISEASES**
PAENIBACILLUS-MUCILAGINOSUS-STAMM ZUR VERWENDUNG ALS DÜNGEMITTEL, ZUR STIMULIERUNG DES WACHSTUMS UND ZUM SCHUTZ VON PFLANZEN VOR PILZERKRANKUNGEN
SOUCHE PAENIBACILLUS MUCILAGINOSUS UTILISÉE EN TANT QU'ENGRAIS ET POUR STIMULER LA CROISSANCE ET EFFECTUER LA PROTECTION DES VÉGÉTAUX CONTRE LES MALADIES À CHAMPIGNONS

(30) Priority: 09.11.2015 RU 2015148020
(43) Date of publication of application: 19.09.2018
(73) Proprietor: Obshchestvo S Ogranichennoi Otvetstvennostiu "Promyshlennye Innovatsii", Moscow 127486 (RU)
(72) Inventor: PLASTININ, Sergey Arkadievich, P. Solovetskiy 164070 (RU); ZDORNOV, Aleksey Vyacheslavovich, Moscow 124482 (RU); NIKULSHIN, Vadim Albertovich, Cheboksary 428010 (RU)
(74) Representative: Icosa
(86) International application number: PCT/RU2015/000929
(87) International publication number: WO 2017/082761

(56) References cited:
- CN-A- 104 480 035
- RU-C1- 2 289 621
- M. MA ET AL: "Complete Genome Sequence of Paenibacillus mucilaginosus 3016, a Bacterium Functional as Microbial Fertilizer", JOURNAL OF BACTERIOLOGY, vol. 194, no. 10, 24 April 2012 (2012-04-24), pages 2777-2778, XP055564912, US ISSN: 0021-9193, DOI: 10.1128/JB.00323-12
- X. F. HU ET AL: "Transfer of Bacillus mucilaginosus and Bacillus edaphicus to the genus Paenibacillus as Paenibacillus mucilaginosus comb. nov. and Paenibacillus edaphicus comb. nov.", INTERNATIONAL JOURNAL OF SYSTEMATIC AND EVOLUTIONARY MICROBIOLOGY, vol. 60, no. 1, 30 July 2009 (2009-07-30), pages 8-14, XP055564913, GB ISSN: 1466-5026, DOI: 10.1099/ijs.0.008532-0
- GOSWAMI D. ET AL.: 'Describing Paenibacillus mucilaginosus strain N3 as an efficient plant growth promoting rhizobacteria (PGPR)' COGENT FOOD & AGRICULTURE vol. 1000714, 23 January 2015, pages 1 - 13, XP055381643

## Description

### FIELD OF THE INVENTION

The invention relates to agricultural microbiology, in particular to the strain of bacteria Paenibacillus mucilaginosus Pm 2906 (VKPM registration number B-12259) used as a fertilizer, a method of stimulating growth and protecting plants from diseases, and the use of Paenibacillus mucilaginosus strain Pm 2906 as a fertilizer and a biological control agent in the prevention and/or treatment of plant diseases.

### BACKGROUND OF THE INVENTION

Currently, the use of bacterial products to improve soil fertility is one of the agrotechnology techniques, an alternative to the increasing use of mineral fertilizers. This is especially relevant due to the required environmental safety.

Bioadditives accelerate the release of nutrients from organic fertilizers, as well as the process of assimilation of the released substances by the plants. In addition, bioadditives contribute to a further accumulation of nutrients in the soil in the form easily digestible by plants.

The use of bacterial fertilizers on the basis of various strains of microorganisms or their metabolites is known.

Thus, the inclusion of an aqueous suspension of bacterial cultures (bacterial stock culture of AMB and azobacterin) into the mixture of peat, lime and mineral additives increases the effectiveness of the fertilizer (Author's cert. 589238).

The inclusion of a suspension of yeast and unicellular algae (author cert. 935501), a consortium of bacteria Streptococcus thermophilus, Streptococcus bovis, Dactobacillus salivaries var salicinicus, Lactobacillus salivaries var salicinicus, Lactobacillus acidophilus (RF Patent 2055823), a suspension of nitrogen-fixing (Azotobacter chroococcum), phosphate-solubilizing (Bacillus mucilaginosus), lactic acid (as a consortium containing Streptococcus thermophilus, Streptococcus bovis, Lactobacillus salivaries var salicinicus, Lactobacillus salivaries var salivarus, Lactobacillus acidophilus (RF Patent 2081866) bacteria into organomineral fertilizers to increase their effectiveness is known.

Fertilizers are known for the preparation of which strains cultivated in nutrient media containing peptone (RF Patent 2241692) or nitrogen compounds (author cert. 1756318) are used, which boosts the vegetative mass of plants in respect to fertilizers based on the strain Bacillus mucilaginosus and Agrobacterium radiobacter, respectively. The application of a biological agent aimed at increasing the yield of agricultural crops and improving the product quality is described, containing strains of such genera as Agrobacterium, Bacillus, Bradyrhizobium, Ervinia, Rhodopseudomonas (RF Patent 2322061) in addition to lactic acid, photosynthetic and nitrogen-containing bacteria.

The prior art describes a fertilizer based on a strain of Bacillus mucilaginosus-Bac 1208, able to produce exopolysaccharide which creates a beneficial microflora of the rhizosphere (RF Patent 2081867), as well as a fertilizer based on a strain of Bacillus mucilaginosus, capable of transforming poorly soluble compounds of phosphorus and potassium into a form accessible to plants, that also has phytopathogenic activity (RF Patent 2408722).

The application of a bioadditive acting as a fertilizer (RF Patent 2148571) is described which consists of associations of nitrogen-fixing bacteria Azotobacter chroococcum and Beijerinckia fluminensis, taken in a ratio (1 - 0.5): (1 - 0.5), as well as bacteria Bacillus megaterium and Bacillus mucilaginosus breaking down phosphorus- and potassium-containing compounds, taken in the ratio (1 - 5 : 0.5 - 2).

The strain of bacteria Azotobacter chroococcum BH-1811 used to produce a biological agent intended to increase the yield of crops and improve their resistance to various diseases is described in RF patent 2289620.

RF patent 2408721 describes the strain of bacteria Agrobacterium radiobacter Ag 1108 having increased nitrogen-fixing properties, as well as a fertilizer on its basis.

A strain of microorganisms Sphingobacterium multivorum is proposed for use in the microbiological industry for obtaining bacterial fertilizers for tomatoes and cucumbers (RF patent 2458119).

The application of a strain of Pseudomonas brassicacearum for the production of a fertilizer is described (RF patent 2453596).

RU 2289621 discloses a strain of bacteria *Bacillus mucilaginosus* for its use in the preparation of bacterial fertilizers and which has a wide spectrum of antagonistic action against fungi and bacteria.

The use of strains of microorganisms, such as Serratia plymuthica (WO/2012.095431), the fungus of the genus Trichoderma (RF patent 2534213), or a composition of Pseudomonas strains mixed with a humate fertilizer and broth culture of Chlorella Vulgaris (RF patent 2291620) for the biological control of plant pathogens is known.

However, known bioadditives used as fertilizers or for protecting plants against phytopathogens have a number of disadvantages due to poor effectiveness and complexity of synthesizing the fertilizer and a narrow spectrum of suppression of phytopathogenic microflora. Moreover, most of the known bioadditives used as fertilizers do not show any antagonistic activity towards phytopathogens. The increase of crop yield with the use of known solutions to a large extent depends on the soil type, the availability of nutrients in it, humus layer thickness and other components.

Thus, the isolation and development of new strains of microorganisms that enhance the effectiveness of organomineral fertilizers and the yield of cultivated plants, with antagonistic activity towards phytopathogens is an important task, the solution of which opens the possibility in principle to achieve on an industrial scale high yields of agricultural crops and high quality of the agricultural products while reducing the amount of fertilizers applied to the soil, as well as to reduce the amount of or eliminate the use of chemicals to protect plants from pathogenic microorganisms.

### SUMMARY OF THE INVENTION

The authors of this invention have proposed for the first time the strain of bacteria Paenibacillus mucilaginosus Pm 2906 (VKPM number B-12259), promoting the formation of metabolic products that accelerate the growth of the root and vegetative system of plants and stimulate the production of phytohormones by plants.

The claimed strain has the ability to dissolve water-insoluble phosphorus and potassium compounds in the soil, and also actively participates in nitrification process.

The use of the claimed strain as a fertilizer makes it possible to significantly reduce the amount of nitrogen, phosphorus and potassium fertilizers applied to the soil.

Paenibacillus mucilaginosus strain Pm 2906 has an enhanced ability to synthesize bacteriocins with a wide spectrum of suppression of phytopathogenic microflora and contributes to increasing stress-resistance, survival and yield of a wide range of agricultural crops and ornamental plants.

In one aspect, the invention is focused on Paenibacillus mucilaginosus strain Pm 2906 (VKPM number B-12259)

In another aspect the invention relates to a method for plant growth stimulation and protection against diseases.

One more aspect of the invention relates to the use of Paenibacillus mucilaginosus strain Pm 2906 as a fertilizer and a biological control agent for the prevention or treatment of plant diseases.

### DETAILED DESCRIPTION OF THE INVENTION

This invention is based on the surprising discovery that Paenibacillus mucilaginosus strain Pm 2906 (VKPM number B-12259) can be used as a highly effective fertilizer stimulating the growth of the plant vegetative mass and root system and increasing the yield of agricultural crops and ornamental plants, and also as a biological control agent in the prevention and treatment of plant diseases.

In accordance with the invention, Paenibacillus mucilaginosus strain Pm 2906 was isolated from cultivated soils by way of a multistage selection method and cultivated on Ashby's medium (Example 1).

### The morphological and cultural characteristics of Paenibacillus mucilaginosus strain Pm 2906:

Growing on Ashby's agar medium (at a temperature of 30 ± 1 °C for 90-100 hours, the colonies are convex or slightly flattened, colorless or light gray, transparent, mucous, with an even edge and a viscous consistency.

Under the microscope, the cells are immobile, large rods of regular shape with rounded ends, single or paired spore-forming, with a mucous membrane.

Cell sizes vary with growth on different nutrient media: 1-1.4 × 4-7 µm. Gram-negative, the Gram staining can vary depending on the stage of the culture development.

### Physiological and biochemical characteristics of Paenibacillus mucilaginosus strain Pm 2906 (VKPM number B-12259):

Paenibacillus mucilaginosus strain Pm 2906 is grown in a fermenter to a concentration of 120 million spores per 1 ml of solution at pH 7.5-8.5 (Example 1).

It absorbs glucose, sucrose, maltose, lactose, arabinose, and hydrolyses starch as a source of carbon. Among alcohols, it absorbs mannitol, glycerin, and lactate.

Ammonium nitrogen, carbamide and nitrates can be used as a nitrogen source.

The strain has the ability to dissolve insoluble and slightly soluble phosphorus and potassium compounds (for example, silicates, apatites, tricalcium phosphate).

In one of the variants of the invention, the fermentation product, which is a suspension of active bacterial cells of Paenibacillus mucilaginosus strain Pm 2906 (VKPM number) with a titer of at least 120 million spores/ml is used as a fertilizer.

The end product remains active for 12 months at a temperature of -3 to +30 °C.

According to the invention, Paenibacillus mucilaginosus strain Pm 2906 is used as a fertilizer for cereals (wheat, barley, oats, rye, rice), potatoes, vegetables, berries, flowers and ornamental plants, legumes, coniferous, deciduous, ornamental trees and shrubs.

The effectiveness of the claimed strain when used as a fertilizer has been studied in the field on various plants.

During field trials of biomass of Paenibacillus mucilaginosus strain Pm 2906 on cherry plum and plum plants using crop treatment at the early stage of the vegetative period, a significant increase in the volume of the root system and plant height was observed (Example 2).

During field trials of biomass of Paenibacillus mucilaginosus strain Pm 2906 on coniferous tree species, an increase in the diameter of the root collar and decrease in the attrition of seedlings were noted (Example 3).

Field trials also demonstrated an increase in potato yield when Paenibacillus mucilaginosus strain Pm 2906 was used as a fertilizer (Example 4).

Another variant involves the use of Paenibacillus mucilaginosus strain Pm 2906 as a biological control agent for the prevention or treatment of plant diseases, particularly rhizoctonia (black scurf), one of the most harmful and common potato diseases.

According to the invention, Paenibacillus mucilaginosus strain Pm 2906 has a negative impact on potato pathogens of fungal origin in open ground (Example 4).

In presowing seed treatment, the preferred consumption rate of the working solution of Paenibacillus mucilaginosus strain Pm 2906 cells at a concentration of 120 million spores/ml is 2.0-4.0 1 per ton of cereal seeds (wheat, barley, oats, rye, rice, buckwheat); from 1.5 to 5 liters per ton of legumes, and from 0.3 to 0.5 liters per ton of potato tubers.

In case of secondary tillage, the preferred consumption rate of the working solution of Paenibacillus mucilaginosus strain Pm 2906 cells at a concentration of 120 million spores/ml for all crops is 0.4 to 1.0 1/ha.

When fertilizing growing plants, the preferred consumption rate of the working solution is 0.4-1.0 1/ha for cereals, 0.4-1.0 1/ha for legumes, from 0.4 to 1.0 1/ha for sugar beet, rice, maize, sunflower, buckwheat, rapeseed, mustard, and potatoes.

When treating fruit and berry, coniferous, deciduous, ornamental trees and shrubs, the pre-sowing treatment of seeds is preferable at the rate of 20-30 ml of Paenibacillus mucilaginosus strain Pm 2906 cells at a concentration of 120 million spores/ml per 200 ml of water. The solution consumption norm is 200 ml per 100 g seeds. When fertilizing young plants and seedlings, it is preferable to irrigate the roots with a solution of 30-50 ml of Paenibacillus mucilaginosus strain Pm 2906 cells at a concentration of 120 million spores/ml per 5-10 1 of water.

The invention is illustrated by the following examples presented to confirm, but not to limit the scope of the claims.

### EXAMPLES

### Example 1. Cultivation of Paenibacillus mucilaginosus strain Pm 2906 (VKPM number B-12259).

A sterile liquid nutrient medium with the following composition, mass %: sucrose-1.0-5.0; K₂HPO₄ - 0.01-0.05; NaCl - 0.01-0.05; MgSO₄ - 0.01-0.05; NH₄NO₃-0.01-0.05; FeSO₄ - 0.0001-0.0007; CaCO₃ - 0.1-0.8; K₂SO₄ - 0.01-0, corn steep 0.1-0.5, is seeded in an inoculator with a cell culture of Paenibacillus mucilaginosus Pm 2906 of February 16, 2009 at a rate of 2-10 % of the volume of the nutrient medium. It is then grown for 12-36 hours at a temperature of 30 ± 5 °C, with continuous stirring and aeration at a rate of 1 volume of air / 1 volume of nutrient medium per minute.

The resulting culture is inoculated into a fermenter with a pre-sterilized liquid nutrient medium with the following composition, mass %: molasses - 0.5-2.0; KH₂PO₄-0.01-0.06; MgSO₄ - 0.005-0.04; NaCl - 0.04-0.08; KNO₃ - 0.005-0.03; CaCO₃-0.1-0.4, corn steep 0.1-0.4, up to 0.5, FeSO4 - 0.0001-0.0007 at a rate of 1-10 % of the volume of the nutrient medium. The culturing is conducted at a temperature of 30 ± 5 °C, with continuous stirring and aeration at a rate of 1 volume of air / 1 volume of nutrient medium per minute. The growth duration is 36-72 hours, pH 7.5-8.5. A slightly viscous liquid culture containing up to 120 million spores/ml is obtained.

The finished fermentation product, which is a suspension of active bacterial cells of Paenibacillus mucilaginosus strain Pm 2906 (VKPM number) with a titer of at least 120 million spores/ml, is used as a fertilizer and a means for the prevention and/or treatment of plant diseases.

The end product remains active for 12 months at a temperature of -3 to +30 °C.

Paenibacillus mucilaginosus strain Pm 2906 when grown by this method shows the ability to actively digest water-insoluble phosphorus and potassium compounds, promote the development of useful microflora of the rhizosphere, increase the production of phytohormones and plant immunity.

Intensive growth of the root system and the vegetative mass of the plant is observed after the application of a fertilizer based on Paenibacillus mucilaginosus strain Pm 2906.

### Example 2. Stimulation of the root system growth in fruit trees

In 2012, an experiment was conducted to test a fertilizer on the basis of Paenibacillus mucilaginosus strain Pm 2906 in Tula region, on the territory of the Scientific Production Center for Biotechnology "Phytogenetics". Biomass of Paenibacillus mucilaginosus strain Pm 2906 (VKPM B-12259) at a concentration of at least 120 million spores/ml was obtained under the conditions described in Example 1.

Cherry plum and plum, grown in pots, were planted in open ground. A month after planting they were treated with the fertilizer. The treatment was performed 3 times every 2 weeks.

The working solution consumption rate (20-30 ml of biomass of Paenibacillus mucilaginosus strain Pm 2906 per 200 ml of water) was 25-50 ml per plant.

As a result of treatment with the fertilizer on the basis of the claimed strain, the volume of the root system increased by 73 % in the Iyulskaya Rosa cherry plum and by 76 % in the Velichavaya plum variety. The height of the fertilized plants increased by an average of 64 % in comparison with the control in the Iyulskaya Rosa variety of cherry plum and 67 % in the Velichavaya plum variety.

### Example 3. Stimulation of survival and development of the root system in coniferous tree species

In 2012, an experiment was conducted on the territory of the Shchelkovo Training and Experimental Forest Range (Moscow Region) to test a fertilizer based on Paenibacillus mucilaginosus strain Pm 2906. Biomass of Paenibacillus mucilaginosus strain Pm 2906 in a concentration of at least 200 million cells/ml was obtained under the conditions described in Example 1.

In a plot of experimental production crops, a single treatment of the root systems of coniferous seedlings with the suspension during planting, as well as fine-droplet spraying of the above-ground part of the plants with the working solution of the product were conducted.

The rate of consumption of the working solution (50 ml of biomass of Paenibacillus mucilaginosus strain Pm 2906 per 10 liters of water) was 2-3 liters of working solution per plant.

The treatment of Swiss pine seedlings with the fertilizer on the basis of *Paenibacillus mucilaginosus* strain showed an increase in the diameter of the root collar by 50-76 % compared to control, and a decrease in the attrition of Norway spruce seedlings by 2-3 %, with the attrition of seedlings in the control of 14 %.

Thus, the application of the fertilizer based on Paenibacillus mucilaginosus strain Pm 2906 stimulates growth of the plant root system mass, increases stress resistance, survival rate and yield of agricultural crops and ornamental plants.

### Example 4. Yield increase

In 2014, field tests of a fertilizer based on the Paenibacillus mucilaginosus strain were conducted in Krasnodar Region (Gulkevichsky district, Gulkevichi, CJSC Plemzavod Gulkevichskiy).

Biomass of the *Paenibacillus mucilaginosus* strain with a titer of at least 120 million spores/ml was obtained under the conditions described in Example 1.

A single presowing treatment of the Grom variety winter wheat seeds was conducted at a rate of bacterial product consumption of 3.0 1 per 1 t of seeds, followed by a single spraying of crops at a rate of 0.4-1.0 1/ha.

At the end of the growing season, the harvest was reaped and the total yield in dt/ha was estimated.

The test results are presented in Table 1.

**Table 1**

| Criterion (ripening stage) | Control | Test |
|---|---|---|
| Flag leaf length, cm | 22.7 | 25.1 |
| Number of ears | 19.0 | 20.1 |
| Ear length, cm | 8.6 | 10.0 |
| Yield, dt/ha | 55.72 | 62.46 |

The data in Table 1 demonstrate that sowing of seeds treated with the fertilizer on the basis of the *Paenibacillus mucilaginosus* strain, with a subsequent treatment of crops during vegetation, resulted in a significant increase in the vegetative mass during the ripening phase, while the yield increase on the experimental plot as compared to the sowing of untreated seeds on the control plot was 11.00 %.

### Example 5. Yield increase and suppression of pathogenic microflora

Biomass of *Paenibacillus mucilaginosus* strain in a concentration of at least 200 million cells/ml was obtained under the conditions described in Example 1.

In 2009, in a field experiment on the territory of the All-Union Scientific and Research Institute for Potato Farming "Korenevo" of Lyubertsy District, Moscow Region, tubers of an early-ripening potato variety (Krepysh) were treated during planting into a soddy-podzolic, sandy loam soil characterized by an acid reaction of the medium and high hydrolytic acidity (pHKCl = 4.47-4.63; Hh = 4.25-4.52 meq / 100 g soil); low total absorbed bases and degree of their saturation (S = 4.2-4.9 meq / 100 g soil; V = 48.5-53.6 %); mobile phosphorus content at the level optimal for potatoes (213-227 mg/kg soil) and an average content of exchangeable potassium (165-192 mg/kg soil); and relatively high humus content (2.39-2.64 % humus).

The planting was done with a cup planter in pre-shaped ridges, planting scheme 70 x 30 cm.

The yield of potatoes with the application of the product based on the strain was 36.3 t/ha against 25 t/ha in the control (the increase compared to control was 45.2 %). Thus, treatment of potato tubers with the product based on the Beijerinckia fluminensis strain Bf 2806 leads to an increase in yield.

The use of the bacterial fertilizer according to the invention had a significant suppressive effect on the spread and development of fungal diseases on potato tubers, the effects of the product application on potato resistance to fungal diseases are presented in Table 2.

**Table 2.**

| Prevalence and development of fungal diseases on potato tubers | | |
|---|---|---|
| Variant | Rhizoctonia | |
| | Prevalence of disease % | Development of disease % |
| Control | 4.2 | 0.7 |
| Test | 0.6 | 0.1 |

In the experiments conducted, the rhizoctonia spread in potato leaves and tubers also dropped, with a disease prevalence of 4.2 % and development rate of 0.7 %.

The aforementioned examples confirm that Paenibacillus mucilaginosus strain Pm 2906, proposed according to the invention, is a highly effective fertilizer that stimulates the growth of the vegetative mass and root system of plants and increases the yield of agricultural crops and ornamental plants, as well as an anti-phytopathogenic agent for the prevention and/or treatment of plant diseases.

## Claims

1. Paenibacillus mucilaginosus strain Pm 2906 deposited in VKPM under number B-12259, used as a fertilizer and biological control agent for the prevention or treatment of fungal disease of plants.

2. Use of Paenibacillus mucilaginosus strain Pm 2906 VKPM B-12259 as a fertilizer

3. Use of Paenibacillus mucilaginosus strain Pm 2906 VKPM B-12259 as a biological control agent for the prevention and/or treatment of fungal disease of plants.

4. Use according to claim **2,** wherein the plant is selected from a group consisting of cereals, potatoes, flowers and ornamental plants, coniferous and deciduous trees.

5. Use according to claim **3** where the disease is rhizoctonia.

6. A method of plant growth stimulation and protection against fungal diseases comprising presowing treatment of soil, seeds or crops with microorganism cells, **characterized in that** a suspension of Paenibacillus mucilaginosus strain Pm 2906 in a concentration of at least 120 million spores/ml is used as a biological control agent.

7. A method of plant growth stimulation and protection against fungal diseases according to claim **6, characterized in that** the rate of working solution consumption per ton of cereal seeds is 2.0-4.0 L; per ton of legumes - from 1.5 to 5 liters, and per ton of potato tubers - from 0.3 to 0.5 liters.

## Patentansprüche

1. *Paenibacillus-Mucilaginosus*-Stamm Pm 2906, hinterlegt in VKPM unter der Nummer B-12259, verwendet als Düngemittel und biologisches Kontrollmittel für die Prävention oder Behandlung von Pilzerkrankungen von Pflanzen.

2. Verwendung des *Paenibacillus-Mucilaginosus*-Stamms Pm 2906 VKPM B-12259 als Düngemittel

3. Verwendung des *Paenibacillus-Mucilaginosus*-Stamms Pm 2906 VKPM B-12259 als biologisches Kontrollmittel für die Prävention und/oder Behandlung von Pilzerkrankungen von Pflanzen.

4. Verwendung nach Anspruch **2,** wobei die Pflanze ausgewählt ist aus einer Gruppe bestehend aus Getreide, Kartoffeln, Blumen und Zierpflanzen, Nadel- und Laubbäumen.

5. Verwendung nach Anspruch **3,** wobei es sich bei der Krankheit um Rhizoctonia handelt.

6. Verfahren zur Stimulierung des Wachstums und zum Schutz von Pflanzen vor Pilzerkrankungen, umfassend Vorsaat-Behandlung von Boden, Samen oder Ernte mit Mikroorganismus-Zellen, **dadurch gekennzeichnet, dass** eine Suspension des *Paenibacillus-Mucilaginosus*-Stamms Pm 2906 in einer Konzentration von wenigstens 120 Millionen Sporen/ml als biologisches Kontrollmittel verwendet wird.

7. Verfahren zur Stimulierung des Wachstums und zum Schutz von Pflanzen vor Pilzerkrankungen nach Anspruch **6, dadurch gekennzeichnet, dass** die Rate von Arbeitslösungsverbrauch pro Tonne Getreidesaat 2,0-4,0 L; pro Tonne Hülsenfrüchte - von 1,5 bis 5 Liter, und pro Tonne Kartoffelknollen - von 0,3 bis 0,5 Liter beträgt.

## Revendications

1. Souche Pm 2906 de *Paenibacillus mucilaginosus* déposée auprès de VKPM sous le numéro B-12259, utilisée comme fertilisant et agent biologique de contrôle pour la prévention ou le traitement de maladie fongique chez les plantes.

2. Utilisation de la souche Pm 2906 VKPM B-12259 de *Paenibacillus mucilaginosus* comme fertilisant.

3. Utilisation de la souche Pm 2906 VKPM B-12259 de *Paenibacillus mucilaginosus* comme agent biologique de contrôle pour la prévention et/ou le traitement de maladie fongique chez les plantes.

4. Utilisation selon la revendication **2** dans laquelle la plante est choisie dans le groupe consistant en les céréales, les pommes de terre, les fleurs et plantes ornementales, les conifères et les arbres à feuilles caduques.

5. Utilisation selon la revendication **3** dans laquelle la maladie est la rhizoctonie.

6. Méthode de stimulation de la croissance de plantes et de protection contre les maladies fongiques comprenant le traitement du sol, des graines ou des cultures avant semis avec des cellules de microorganisme, **caractérisée en ce qu'**une suspension de concentration d'au moins 120 millions de spores/ml de la souche Pm 2906 de *Paenibacillus mucilaginosus* est utilisé comme agent biologique de contrôle.

7. Méthode de stimulation de la croissance de plantes et de protection contre les maladies fongiques selon la revendication **6, caractérisée en ce que** le taux de consommation de la solution soit de 2,0 à 4,0 litres par tonne de graines de céréales, de 1,5 à 5 litres par tonne de légumes et de 0,3 à 0,5 litres par tonne de tubercules de pomme de terre.
